# EUROPÄISCHE PATENTSCHRIFT

(11) **EP 0 652 903 B1**
(45) Veröffentlichungstag und Bekanntmachung des Hinweises auf die Patenterteilung: **04.03.1998**
(21) Anmeldenummer: 92900077.6
(22) Anmeldetag: 14.11.1991
(51) Int. Cl.: C07K 14/00, C12N 15/23, A61K 38/21

(54) **NEUES MENSCHLICHES, REKOMBINANTES INTERFERON GAMMA**
NEW HUMAN RECOMBINANT $g(g) INTERFERON
NOUVEL INTERFERON GAMMA HUMAIN RECOMBINE

(30) Priorität: 19.11.1990 DE 4036856
(43) Veröffentlichungstag der Anmeldung: 17.05.1995
(73) Patentinhaber: FRAUNHOFER-GESELLSCHAFT ZUR FÖRDERUNG DER ANGEWANDTEN FORSCHUNG E.V., 80636 München (DE)
(72) Erfinder: SLODOWSKI, Otto, D-3000 Hannover 61 (DE); BÖHM, Joachim, D-3000 Hannover 61 (DE); OTTO, Bernd, D-3000 Hannover 51 (DE)
(86) Internationale Anmeldenummer: DE9100912
(87) Internationale Veröffentlichungsnummer: WO9208737

(56) Entgegenhaltungen:
- EP-A- 0 146 354
- EP-A- 0 166 993
- EP-A- 0 170 917
- EP-A- 0 256 424
- EP-A- 0 306 870

## Beschreibung

### Technisches Gebiet

Die Erfindung betrifft eine neue Mutante des Interferon-γ, DNA-Sequenzen und eine Plasmid-DNA, die für diese Mutante des Interferon-γs kodieren sowie die Verwendung der Mutante für medizinische Zwecke.

### Stand der Technik

Die Interferone werden in 3 Klassen eingeteilt und zwar in Interferon-α, Interferon-β und Interferon-γ.

Vor allem das Interferon-γ hat in jüngster Zeit durch seine Anwendung als Therapeutikum große Bedeutung gewonnen. Dies war vor allem dadurch möglich, daß es gelungen ist, Interferon-γ auf gentechnologischem Wege (sog. rekombinantes Interferon-γ) herzustellen.

Das auf gentechnologischem Wege hergestellte Interferon-γ enthält 144 Aminosäuren und damit eine Aminosäure, nämlich Methionin in Pos. 0, mehr als natürliches Interferon-γ mit 143 Aminosäuren.

In EP-A-0 306 870 werden Peptide beschrieben, die sich von Interferon-γ durch Abspaltung von 7 bis 11 Aminosäuren am C-terminalen Ende unterscheiden.

Aufgrund der systemischen Applikation werden jedoch Interferone mit unphysiologisch hohen Konzentrationen verabreicht. Diese hohen Konzentrationen stellen hohe Anforderungen an die Formulierung der Interferone, die zudem zu einer Antigenität beitragen können.

Zur Verbesserung der therapeutischen Anwendung wäre es deshalb äußerst nützlich, wenn eine Mutante von Interferon-γ zur Verfügung stehen würde, die eine erhöhte Aktivität aufweist, so daß dann bei der Applikation mit geringeren Konzentrationen gearbeitet werden könnte. Weiterhin wäre es wünschenswert, daß diese Mutante des Interferon-γ in einer möglichst hohen Expressionsrate anfällt.

Aufgabe der Erfindung ist es, eine neue Mutante des Interferon-γs anzugeben, die gegenüber den bisherigen rekombinanten Interferon-γ mit 144 Aminosäuren eine wesentlich höhere Aktivität aufweist. Aufgabe der Erfindung ist es weiterhin, DNA-Sequenzen und eine Plasmid-DNA anzugeben, die für diese Mutante kodieren, und ein Verfahren aufzuzeigen, das es ermöglicht, daß die neue Mutante in einer möglichst hohen Ausbeute anfällt.

### Darstellung der Erfindung

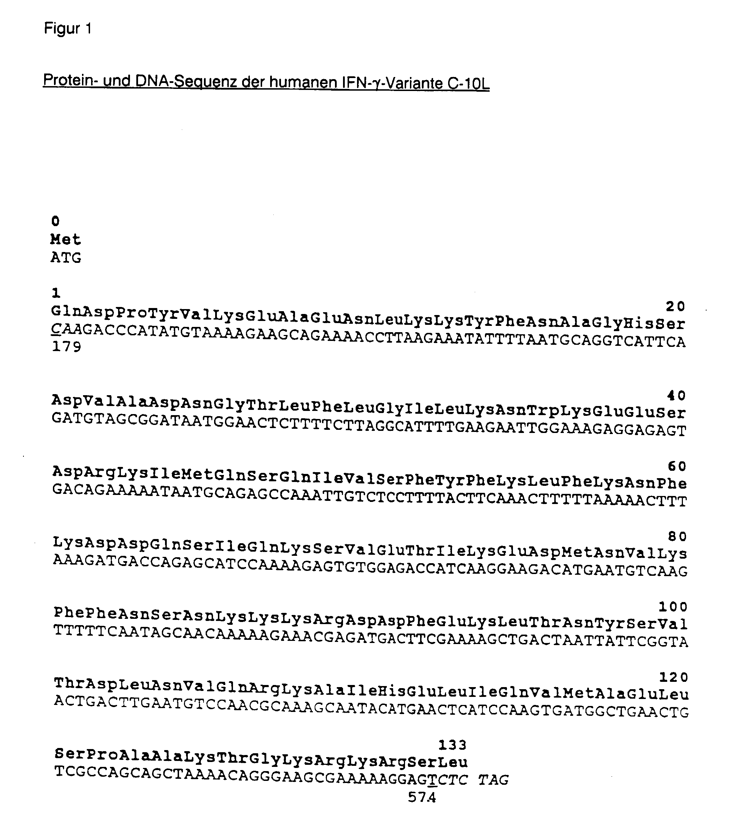

Die Aufgabe wird dadurch gelöst, daß DNA-Sequenzen und eine Plasmid-DNA vorgeschlagen werden, die für eine Aminosäuresequenz kodieren, die gegenüber dem bisherigen rekombinanten IFN-γ eine wesentlich erhöhte Aktivität aufweist. Dieses neue Polypeptid (im folgenden als Interferon-γ C-10L bezeichnet) enthält 134 Aminosäuren. Die Aminosäuren 1 bis 132 entsprechen dabei denen des natürlichen Interferon-γs. Die erste Aminosäure, Methionin in Position Null, ist zusätzlich vorhanden und wurde durch Proteinsequenzierung nachgewiesen. Die Aminosäure in Position 133 ist Leucin statt Glutamin. Die vollständige DNA- und Proteinsequenz ist in Figur 1 wiedergegeben. Gleichzeitig wird ein Verfahren angegeben, das es erlaubt, die Mutante in hohen Ausbeuten zu erhalten. Erfindungsgemäß wird vorgeschlagen, die Reinigung in einem sog. Batch-Verfahren durchzuführen.

Überraschenderweise wurde nun gefunden, daß das IFN-γ C-10L eine um das 4-fach höhere Aktivität als das komplette 143 Aminosäure lange IFN-γ hat. Das IFN-γ C-10L hat zudem eine um den Faktor 24 höhere antiproliferative Aktivität als das IFN-γ und zwar auf humanen WISH-Zellen. Mit dieser gegenüber dem bekannten IFN-γ um 10 Aminosäuren verkürzten Mutante steht damit erstmals ein Interferon-γ zur Verfügung, das aufgrund seiner hohen Aktivität und seiner hohen Expressionsrate eine gezieltere und niedrigere Dosierung bei der Verwendung als Therapeutikum erlaubt. Aufgrund der hohen Expressionsrate ist es weiterhin möglich, dieses Interferon-γ auch als Feinchemikalie für in vitro-Versuche zu verwenden, z.B. als Standard für Interferonspiegelmessungen.

Im folgenden wird die neue Mutante sowie die Herstellung detailliert beschrieben.

Die Herstellung des Interferon-γ C-10L erfolgt durch Herausschneiden eines Teils des Gens und Einsetzen bzw. Einpassen in eine Plasmid-DNA hinter einem Regulationsbereich sowie Transfektion von Bakterienzellen mit dieser DNS. In einem weiteren Schritt wird IFN-γ C-10L in einem Kationenaustauscherprozeß konzentriert und in einem zweiten Schritt einer Hochreinigung unterzogen.

Im folgenden wird beispielhaft die Herstellung der Plasmid-DNA (Hinterlegungsnummer DSM 6238) beschrieben, die für die Interferon-γ-Variante IFN-γ C-10L kodiert.

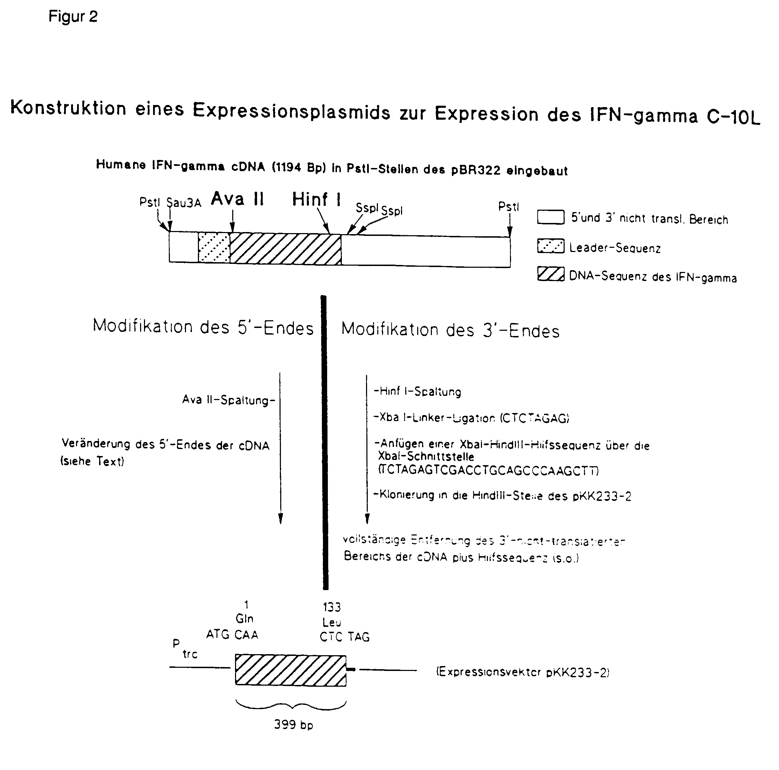

Die Einzelschritte sind in Figur 2 dargestellt.

Als Ausgangsmaterial wurde eine cDNA verwendet, die mit Standardmethoden aus menschlichen Zellen gewonnen wurde. Diese cDNA wurde sequenziert und ist ohne Poly-A 1194 Basenpaare (Bp) lang. Sie enthält den gesamten kodierenden Bereich sowie 5' und 3' nicht translatierte Sequenzen. Für die Konstruktion des Expressionsplasmides wurden die Nukleotide 182 bis 574 verwendet.

Der 5' nicht translatierte Bereich (1 - 109) sowie die Leadersequenz des Proteins (110 - 181) wurden durch Spaltung mit der Restriktionsendonuklease Ava II (Erkennungssequenz GGA/TCC. 181 - 185) entfernt. Das Initiationscodon ATG (für Met) sowie das Codon für die erste Aminosäure wurden durch synthetische DNA-Stücke (kommerziell erhältliche Linker) dem 5'-Ende der cDNA hinzugefügt. Dabei wurde das natürliche Codon CAG (für Gln) durch CAA (ebenfalls für GIn) ersetzt. Die dafür nötigen Einzelschritte sind allgemeiner Stand der Technik.

Der 3' nicht translatierte Bereich sowie ein Teil des codierenden Bereichs der cDNA wurden durch Spaltung mit der Restriktionsendonuklease Hinf I (Erkennungssequenz GANTC, 571 - 575) entfernt. Die Ligation eines Linkers mit der Erkennungssequenz für Xba I (CTCTAGAG) liefert das Codon für Aminosäure 133 (Leu) sowie das Stopcodon (TAG). Die verkürzte cDNA wurde durch Ligation einer Hilfssequenz in den Expressionsvektor eingefügt.

Für die Expression von Interferon-γ in E. coli wurde das Plasmid pKK233-2. konstruiert von E. Amann und J. Brosius (Gene 40 (1985) 1893 - 190) verwendet. Es besitzt den induzierbaren trc-Promotor, eine multiple cloning site, die das Startcodon (ATG) enthält sowie Terminatoren für die RNA-Polymerase.

Diese Plasmid-DNA (Hinterlegungsnummer DSM 6238) stellt dann das Ausgangsmaterial zur Gewinnung des IFN-γ C-10L dar.

Das IFN-γ C-10L wird in Bakterienzellen (JM 105) mit einer Rate von 30% des totalen Proteins exprimiert. Zu über 90% wird dieses IFN-γ in Form von unlöslichen "Inclusion bodies" abgelagert.

Für die Reinigung dieses IFN-γ werden Bakterienzellen nach erfolgreicher Expression aufgebrochen und die "Inclusion bodies" durch mehrfaches Waschen von löslichen bakteriellen Proteinen befreit. Das Aufbrechen wird bevorzugt mechanisch; insbesonders durch Ultraschall vorgenommen. Die "Inclusion bodies" und damit das IFN-γ werden durch einen Denaturierungsschritt mit Guanidiniumchlorid in Lösung gebracht; in einem Renaturierungsschritt durch Verdünnen in einen Phosphatpuffer wird das IFN-γ in die biologisch aktive Form gefaltet. Das dadurch zu mehr als 90% saubere IFN-γ wird durch einen Kationenaustauscherprozeß im "Batch-Verfahren" konzentriert und weiter gereinigt und erreicht durch einen weiteren Gelfiltrationsschritt einen Reinheitsgrad von mehr als 95%.

Unter einem Batch-Verfahren im Sinne dieser Erfindung wird folgendes verstanden: Das Kationenaustauschermaterial wird gleichmäßig so in einer Interferon-γ-Lösung eingerührt, daß das Interferon-γ gleichmäßig verteilt an alles Kationenaustauschermaterial bindet und die Interferon-γ-Protein-Konzentration nicht mehr als ca. 2 mg/ml gepacktes Kationenaustauschermaterial beträgt. Dieses Verfahren wird dann als Batch-Verfahren bezeichnet. Das mit Interferon-γ beladene Kationenaustauschermaterial wird auch im Batch mit Phosphatpuffer gewaschen und das Interferon-γ dann im Batch mit Kochsalzlösung in Phosphatpuffer eluiert. Als Kationenaustauscher können hier z.B. Cellulose oder Affi-Gel-Blue angewendet werden.

Dieses Batch-Verfahren bietet entscheidende Vorteile und führt zu einer Ausbeutesteigerung bei der Reinigung von IFN-γ auf 40%, verglichen mit 10% beim herkömmlichen Säulenverfahren.

Dieser Kationenaustauscherprozeß wird nämlich normalerweise mit einer Säulenchromatographie durchgeführt, d.h. das Interferon-γ wird nach dem Renaturierungsschritt in Gegenwart von z.B. 0,2 M Guanidiniumchlorid und einem Phosphatpuffer auf eine Säule mit dem Kationenaustauschermaterial gepumpt und bindet wegen der hohen Bindungskapazität des Kationenaustauschermaterials für das Interferon-γ mit dementsprechend hoher Konzentration im oberen Teil der Säule. Wegen der hohen Konzentration des gebundenen Interferon-γ sind die Elutionsausbeuten sehr gering, d.h. nur ein kleiner Teil des gebundenen Interferon-γ kann mit entsprechenden Salzlösungen von dem Kationenaustauschermaterial abgelöst werden. Demnach bietet das erfindungsgemäße Verfahren gegenüber dem Stand der Technik entscheidende Vorteile.

Figur 1 zeigt nun die Protein- und DNA-Sequenz der humanen Interferon-γ-Variante C-10L.

Danach enthält die Interferon-γ-Mutante C-10L 134 Aminosäuren. Die erste Aminosäure (Methionin in Position Null) ist dabei zusätzlich vorhanden und wurde durch ProteinSequenzierung bestätigt. Die Aminosäuren 1 bis 132 entsprechen denen des natürlichen Interferon-γ. Die Aminosäure in Position 133 ist Leucin statt Glutamin. Die korrekte Abfolge der Nukleotide der kodierenden Sequenz sowie der flankierenden Bereiche wurden durch DNA-Sequenzierung bestätigt.

Das Interferon-γ C-10L hat ein Molekulargewicht von ca. 30 000 daltons unter nativen Bedingungen und einen S-Wert von 2,5, d.h. es ist in einer dimeren Form organisiert. Unter denaturierenden Bedingungen in SDS zeigt es als ein Monomer ein MW von ca. 15 000 daltons. Der isoelektrische Punkt wurde mit 10,0 gemessen.

Das Interferon-γ C-10L hat eine spezifische antivirale Aktivität von 8 x 10⁷ u/mg Protein (gemessen auf humanen Lungenfibroblastenkarzinomzellen A 549 mit dem EMC-Virus) und ist damit 4-fach aktiver als das komplette 143 Aminosäuren lange Interferon-γ.

Das Interferon-γ C-10L hat eine um den Faktor 24 höhere antiproliferative Aktivität als das Interferon-γ und zwar auf humanen WISH-Zellen.

Das Interferon-γ C-10L induziert die Expression des MHC Klasse II Antigens HLA-DR auf humanen Colon-Carcinomzellen um den Faktor 8 besser als das Interferon-γ. Erstaunlicherweise ist die Rezeptorbindung des Interferon-γ C-10L auf diesen Colonzellen jedoch um den Faktor 2 schlechter als die entsprechende Rezeptorbindung des Interferon-γ. Gemessen wurde die Rezeptorbindung mit 32P markiertem Interferon-γ in Kompetitionsversuchen, in denen 32P Interferon-γ mit nicht markiertem Interferon-γ oder Interferon-γ C-10L kompetiert wird und "vice versa" 32P Interferon-γ C-10L mit nicht markiertem Interferon-γ bzw. Interferon-γ.

In dieser wichtigen Eigenschaft, der Rezeptorbindung, unterscheidet sich das Interferon-γ C-10L von einem von der Garotta-Gruppe beschriebenen Interferon-γ, das am COOH-Ende ebenfalls um 10 Aminosäuren verkürzt ist, das aber als endständige Aminosäure ein Glutamin an Stelle des Leucins in dem Interferon-γ C-10L trägt und eine 4-fach bessere Rezeptorbindung als Interferon-γ zeigt.

Durch die hier gezeigte Interferon-γ-Variante C-10L steht demnach erstmals eine Mutante des Interferon-γ zur Verfügung, die eine erhöhte Aktivität aufweist und die zugleich in erhöhter Ausbeute hergestellt werden kann.

## Patentansprüche

1. Polypeptid IFN-γ C-10L mit folgender Aminosäuresequenz:

0

ATG

1                                                                                    20

CAAGACCCATATGTAAAAGAAGCAGAAAACCTTAAGAAATATTTTAATGCAGGTCATTCA
179

                                                                                     40

GATGTAGCGGATAATGGAACTCTTTTCTTAGGCATTTTGAAGAATTGGAAAGAGGAGAGT

                                                                                     60

GACAGAAAAATAATGCAGAGCCAAATTGTCTCCTTTTACTTCAAACTTTTTAAAAACTTT

                                                                                     80

AAAGATGACCAGAGCATCCAAAAGAGTGTGGAGACCATCAAGGAAGACATGAATGTCAAG

                                                                                    100

TTTTTCAATAGCAACAAAAAGAAACGAGATGACTTCGAAAAGCTGACTAATTATTCGGTA

                                                                                    120

ACTGACTTGAATGTCCAACGCAAAGCAATACATGAACTCATCCAAGTGATGGCTGAACTG

                                                    133

TCGCCAGCAGCTAAAACAGGGAAGCGAAAAAGGAGTCTC TAG
                                                574

2. DNA-Sequenz, die für ein Polypeptid mit der folgenden Aminosäuresequenz kodiert:

0

ATG

1                                                                    20

CAAGACCCATATGTAAAAGAAGCAGAAAACCTTAAGAAATATTTTAATGCAGGTCATTCA
179

                                                                     40

GATGTAGCGGATAATGGAACTCTTTTCTTAGGCATTTTGAAGAATTGGAAAGAGGAGAGT

                                                                     60

GACAGAAAAATAATGCAGAGCCAAATTGTCTCCTTTTACTTCAAACTTTTTAAAAACTTT

                                                                     80

AAAGATGACCAGAGCATCCAAAAGAGTGTGGAGACCATCAAGGAAGACATGAATGTCAAG

                                                                    100

TTTTTCAATAGCAACAAAAAGAAACGAGATGACTTCGAAAAGCTGACTAATTATTCGGTA

                                                                    120

ACTGACTTGAATGTCCAACGCAAAGCAATACATGAACTCATCCAAGTGATGGCTGAACTG

                                          133

TCGCCAGCAGCTAAAACAGGGAAGCGAAAAAGGAGTCTC TAG
                                      574

3. Plasmid-DNA mit der Hinterlegungsnummer DSM 6238, die für folgende Aminosäuresequenz kodiert:

0

ATG

1
20

CAAGACCCATATGTAAAAGAAGCAGAAAACCTTAAGAAATATTTTAATGCAGGTCATTCA
179

40

GATGTAGCGGATAATGGAACTCTTTTCTTAGGCATTTTGAAGAATTGGAAAGAGGAGAGT

60

GACAGAAAAATAATGCAGAGCCAAATTGTCTCCTTTTACTTCAAACTTTTTAAAAACTTT

80

AAAGATGACCAGAGCATCCAAAAGAGTGTGGAGACCATCAAGGAAGACATGAATGTCAAG

100

TTTTTCAATAGCAACAAAAAGAAACGAGATGACTTCGAAAAGCTGACTAATTATTCGGTA

120

ACTGACTTGAATGTCCAACGCAAAGCAATACATGAACTCATCCAAGTGATGGCTGAACTG

133

TCGCCAGCAGCTAAAACAGGGAAGCGAAAAAGGAGTCTC TAG
574

4. Herstellung der Plasmid-DNA nach Anspruch 3, dadurch gekennzeichnet, daß der 5' nicht translatierte sowie ein Teil des kodierenden Bereiches einer cDNA mit 1194 Bp und 143 Aminosäuren durch Spaltung mit einer Restriktionsendonuklease entfernt wird und daß der 3' nicht translatierte Bereich sowie ein Teil des kodierten Bereiches der cDNA ebenfalls durch Spaltung mit einer Restriktionsendonuklease entfernt wird und daß dieses Gen in dem Plasmid PKK 233-2 in Bakterienzellen eingeführt wird.

5. Herstellung nach Anspruch 4, dadurch gekennzeichnet, daß die Bakterienzelle E. coli ist.

6. Herstellung nach Anspruch 4 und 5, dadurch gekennzeichnet, daß die Restriktionsendonuklease zur Abspaltung des 5' Bereiches und der Leadersequenz Ava II ist.

7. Herstellung der Plasmid-DNA nach Anspruch 4 bis 6, dadurch gekennzeichnet, daß Restriktionsendonuklease zur Abspaltung des 3' nicht translatierten Bereiches sowie eines Teils des kodierenden Bereiches die Restriktionsendonuklease Hinf I ist.

8. Herstellung des Polypeptides nach Anspruch 1 gekennzeichnet durch die Kombination folgender Merkmale, daß
a) das IFN-γ C-10L aus der Plasmid-DNA nach Anspruch 3 exprimiert und daß
b) die Bakterienzellen nach erfolgreicher Expression aufgebrochen und die erhaltenen Inclusion bodies durch Waschen von löslichen bakteriellen Proteinen befreit werden und daß
c) die Inclusion bodies durch eine Denaturierung in Lösung gebracht und anschließend einem Renaturierungsschritt unterzogen werden und daß
d) das Interferon-γ C-10L gereinigt wird.

9. Herstellung nach Anspruch 8, dadurch gekennzeichnet, daß das Interferon-γ C-10L durch einen Kationenaustauscherprozeß im Batch-Verfahren konzentriert und anschließend durch eine Gelfiltration hochgereinigt wird.

10. Herstellung nach Anspruch 8 und 9, dadurch gekennzeichnet, daß die Bakterienzellen mechanisch, z. B. mit Ultraschall, aufgebrochen werden.

11. Herstellung nach Anspruch 8 bis 10, dadurch gekennzeichnet, daß beim Batch-Verfahren das Kationenaustauschermaterial in gleichmäßigen Kontakt mit der IFN-γ C-10L-Lösung gebracht wird, um eine gleichmäßige Belegung des Harzes zu erreichen, und daß anschließend im Batch gewaschen und eluiert wird.

12. Herstellung nach Anspruch 8 bis 11, dadurch gekennzeichnet, daß als Kationenaustauscher übliche Materialien, wie z.B. CM-Cellulose oder Affi-Gel-Blue, verwendet wird.

13. Polypeptid nach Anspruch 1 zur Verwendung als Medikament.

14. Polypeptid nach Anspruch 13 zur Verwendung als Medikament zur Bekämpfung von Rheuma und Nierenkrebs.

15. Verwendung des Polypeptides nach Anspruch 1 als Feinchemikalie für in vitro-Versuche, z.B. für Interferonspiegelmessung.

## Claims

1. Polypeptide IFN-γ C-1OL with the following amino acid sequence:

0

ATG

1
 20
CAAGACCCATATGTAAAAGAAGCAGAAAACCTTAAGAAATATTTTAATGCAGGTCATTCA
179

 40
GATGTAGCGGATAATGGAACTCTTTTCTTAGGCATTTTGAAGAATTGGAAAGAGGAGAGT

 60
GACAGAAAAATAATGCAGAGCCAAATTGTCTCCTTTTACTTCAAACTTTTTAAAAACTTT

 80
AAAGATGACCAGAGCATCCAAAAGAGTGTGGAGACCATCAAGGAAGACATGAATGTCAAG

 100
TTTTTCAATAGCAACAAAAAGAAACGAGATGACTTCGAAAAGCTGACTAATTATTCGGTA

 120
ACTGACTTGAATGTCCAACGCAAAGCAATACATGAACTCATCCAAGTGATGGCTGAACTG

133

TCGCCAGCAGCTAAAACAGGGAAGCGAAAAAGGAGTCTC TAG
574

2. DNA sequence which codes for a polypeptide with the following amino acid sequence:

0

ATG

1
20

CAAGACCCATATGTAAAAGAAGCAGAAAACCTTAAGAAATATTTTAATGCAGGTCATTCA
179

40

GATGTAGCGGATAATGGAACTCTTTTCTTAGGCATTTTGAAGAATTGGAAAGAGGAGAGT

60

GACAGAAAAATAATGCAGAGCCAAATTGTCTCCTTTTACTTCAAACTTTTTAAAAACTTT

80

AAAGATGACCAGAGCATCCAAAAGAGTGTGGAGACCATCAAGGAAGACATGAATGTCAAG

100

TTTTTCAATAGCAACAAAAAGAAACGAGATGACTTCGAAAAGCTGACTAATTATTCGGTA

120

ACTGACTTGAATGTCCAACGCAAAGCAATACATGAACTCATCCAAGTGATGGCTGAACTG

133

TCGCCAGCAGCTAAAACAGGGAAGCGAAAAAGGAGTCTC TAG
574

3. Plasmid DNA with the deposit number DSM 6238, which codes for the following amino acid sequence:

0

ATG

1                                                                    20

CAAGACCCATATGTAAAAGAAGCAGAAAACCTTAAGAAATATTTTAATGCAGGTCATTCA
179

                                                                     40

GATGTAGCGGATAATGGAACTCTTTTCTTAGGCATTTTGAAGAATTGGAAAGAGGAGAGT

                                                                     60

GACAGAAAAATAATGCAGAGCCAAATTGTCTCCTTTTACTTCAAACTTTTTAAAAACTTT

                                                                     80

AAAGATGACCAGAGCATCCAAAAGAGTGTGGAGACCATCAAGGAAGACATGAATGTCAAG

                                                                    100

TTTTTCAATAGCAACAAAAAGAAACGAGATGACTTCGAAAAGCTGACTAATTATTCGGTA

                                                                    120

ACTGACTTGAATGTCCAACGCAAAGCAATACATGAACTCATCCAAGTGATGGCTGAACTG

                                          133

TCGCCAGCAGCTAAAACAGGGAAGCGAAAAAGGAGTCTC TAG
                                      574

4. Manufacture of the plasmid DNA according to claim 3, characterized in that the 5' non-translated region as well as a part of the coded region of a cDNA with 1 194 base pairs and 143 amino acids is removed by cleaving with a restriction endonuclease and in that the 3' non-translated region as well as a part of the coded region of the cDNA is also removed by cleaving with a restriction endonuclease and in that this gene is inserted into the plasmid PKK 233-2 in bacteria cells.

5. Manufacture according to claim 4, characterized in that the bacteria cell is E. coli.

6. Manufacture according to claim 4 or 5, characterized in that the restriction endonuclease for cleaving the 5' region and the leader sequence is Ava II.

7. Manufacture of the plasmid DNA according to claim 4 to 6, characterized in that the restriction endonuclease for cleaving the 3' non-translated region as well as a part of the coded region is the restriction endonuclease Hinf I.

8. Manufacture of the polypeptide according to claim 1, characterized by the combination of the following features,
a) the IFN-γ C-10L is expressed from the plasmid DNA according to claim 3 and
b) the bacteria cells are broken up after completed expression and the inclusion bodies which are obtained are freed from soluble bacterial proteins by washing and
c) the inclusion bodies are brought into solution by denaturation and then subjected to a renaturation step and
d) the interferon-γ C-10L is purified.

9. Manufacture according to claim 8, characterized in that the interferon-γ C-10L is concentrated by a cation exchange process in a batch method and is then highly purified by a gel filtration.

10. Manufacture according to claim 8 or 9, characterized in that the bacteria cells are broken up mechanically, e.g. by ultra-sound.

11. Manufacture according to claim 8 to 10, characterized in that the cation exchange material is brought into uniform contact with the IFN-γ C-10L solution in the batch method, in order to obtain uniform coating of the resin, and in that it is then washed in the batch and eluted.

12. Manufacture according to claim 8 to 11, characterized in that customary materials are used as cation exchangers, such as CM-cellulose or Affi-gel blue for example.

13. A polypeptide according to claim I for use as a medicament.

14. A polypeptide according to claim 13 for use as a medicament for combatting rheumatism and cancer of the kidney.

15. Use of the polypeptide according to claim 1 as a fine chemical for in vitro research, e.g. for interferon mirror measurement.

## Revendications

1. Polypeptide IFN-γ C-10L ayant la séquence d'aminoacides suivante :

0

ATG

1                                                                    20

CAAGACCCATATGTAAAAGAAGCAGAAAACCTTAAGAAATATTTTAATGCAGGTCATTCA
179

                                                                     40

GATGTAGCGGATAATGGAACTCTTTTCTTAGGCATTTTGAAGAATTGGAAAGAGGAGAGT

                                                                     60

GACAGAAAAATAATGCAGAGCCAAATTGTCTCCTTTTACTTCAAACTTTTTAAAAACTTT

                                                                     80

AAAGATGACCAGAGCATCCAAAAGAGTGTGGAGACCATCAAGGAAGACATGAATGTCAAG

                                                                    100

TTTTTCAATAGCAACAAAAAGAAACGAGATGACTTCGAAAAGCTGACTAATTATTCGGTA

                                                                    120

ACTGACTTGAATGTCCAACGCAAAGCAATACATGAACTCATCCAAGTGATGGCTGAACTG

                                       133

TCGCCAGCAGCTAAAACAGGGAAGCGAAAAAGGAGTCTC TAG
                                    574

2. Séquence d'ADN qui code pour un polypeptide ayant une séquence d'aminoacides, suivante :

0

ATG

1
20

CAAGACCCATATGTAAAAGAAGCAGAAAACCTTAAGAAATATTTTAATGCAGGTCATTCA
179

40

GATGTAGCGGATAATGGAACTCTTTTCTTAGGCATTTTGAAGAATTGGAAAGAGGAGAGT

60

GACAGAAAAATAATGCAGAGCCAAATTGTCTCCTTTTACTTCAAACTTTTTAAAAACTTT

80

AAAGATGACCAGAGCATCCAAAAGAGTGTGGAGACCATCAAGGAAGACATGAATGTCAAG

100

TTTTTCAATAGCAACAAAAAGAAACGAGATGACTTCGAAAAGCTGACTAATTATTCGGTA

120

ACTGACTTGAATGTCCAACGCAAAGCAATACATGAACTCATCCAAGTGATGGCTGAACTG

133

TCGCCAGCAGCTAAAACAGGGAAGCGAAAAAGGAGTCTC TAG
574

3. ADN de plasmide ayant le numéro de dépôt DSM 6238 qui code pour la séquence d'aminoacides suivante :

0

ATG

1

20
CAAGACCCATATGTAAAAGAAGCAGAAAACCTTAAGAAATATTTTAATGCAGGTCATTCA
179


40
GATGTAGCGGATAATGGAACTCTTTTCTTAGGCATTTTGAAGAATTGGAAAGAGGAGAGT


60
GACAGAAAAATAATGCAGAGCCAAATTGTCTCCTTTTACTTCAAACTTTTTAAAAACTTT


80
AAAGATGACCAGAGCATCCAAAAGAGTGTGGAGACCATCAAGGAAGACATGAATGTCAAG


100
TTTTTCAATAGCAACAAAAAGAAACGAGATGACTTCGAAAAGCTGACTAATTATTCGGTA


120
ACTGACTTGAATGTCCAACGCAAAGCAATACATGAACTCATCCAAGTGATGGCTGAACTG

133

TCGCCAGCAGCTAAAACAGGGAAGCGAAAAAGGAGTCTC TAG
574

4. Fabrication de l'ADN de plasmide selon la revendication 3,
caractérisée en ce que
la zone en 5' non translatée ainsi qu'une partie de la zone codante de l'ADNc avec 1194 pb et 143 aminoacides, est éliminée par clivage avec une endonucléase de restriction et en ce que la zone en 3' non translatée ainsi qu'une partie de la zone codée de l'ADNc est également éliminée par clivage avec une endonucléase de restriction et en ce que ce gène est introduit dans le plasmide PKK 233-2 dans les cellules bactériennes.

5. Fabrication selon la revendication 4,
caractérisée en ce que
les cellules bactériennes sont E. Coli.

6. Fabrication selon les revendications 4 et 5,
caractérisée en ce que
l'endonucléase de restriction pour la coupure de la zone en 5' et de la séquence de leader est Ava II.

7. Fabrication de l'ADN de plasmide selon les revendications 4 à 6,
caractérisée en ce que
l'endonucléase de restriction pour la coupure de la zone en 3' non translatée ainsi que d'une partie de la zone codante, est l'endonucléase de restriction Hinf I

8. Fabrication du polypeptide selon la revendication 1,
caractérisée en ce que
a) l'IFN-γ C-10L est exprimé à partir de l'ADN de plasmide selon la revendication 3 et que,
b) les cellules bactériennes sont désintégrées après expression fructueuse et les corps (bodies) d'inclusion sont débarrassés par lavage, de protéines bactériennes solubles et en ce que
c) les corps d'inclusion sont mis en solution par une dénaturation et sont soumis ensuite à une étape de renaturation et en ce que,
d) l'interféron-γ C-10L est purifié.

9. Fabrication selon la revendication 8,
caractérisée en ce que
l'interféron-γ C-10L est concentré au moyen d'un processus d'échangeur de cations dans un procédé par lots et ensuite est amené à une purification poussée par filtration sur gel.

10. Fabrication selon les revendications 8 et 9,
caractérisée en ce que
les cellules bactériennes sont dissociées mécaniquement par exemple avec les ultrasons.

11. Fabrication selon les revendications 8 à 10,
caractérisée en ce que
dans le procédé par lot, le matériau d'échangeur de cations est amené en contact régulier avec la solution d'IFN-γ C-10L afin d'obtenir un dépôt régulier de la résine et en ce qu'ensuite on lave en lot et on élue.

12. Fabrication selon les revendications 8 à 11,
caractérisée en ce que
comme échangeur de cations on utilise des matériaux usuels comme par exemple la CM-cellulose ou le Gel-Affi-Bleu.

13. Polypeptide selon la revendication 1 pour l'utilisation comme médicament.

14. Polypeptide selon la revendication 13 pour l'utilisation comme médicament pour combattre les rhumatismes et les cancers rénaux.

15. Utilisation du polypeptide selon la revendication 1 comme produit chimique fin pour des essais in vitro par exemple pour la mesure des taux d'interféron.
